# EUROPÄISCHE PATENTANMELDUNG

(11) **EP 4 011 268 A1**
(43) Veröffentlichungstag der Anmeldung: **15.06.2022**
(21) Anmeldenummer: 21210416.0
(22) Anmeldetag: 25.11.2021
(51) Int. Cl.: A61B 1/00, A61B 1/015, A61B 1/06, A61B 1/12, A61B 1/05

(54) **HYBRIDENDOSKOP MIT ROTATIONSTROMMEL FÜR STERILE MEDIZINISCHE ANWENDUNGEN**

(30) Priorität: 09.12.2020 DE 102020132776
(71) Anmelder: Karl Storz SE & Co. KG, 78532 Tuttlingen (DE)
(72) Erfinder: Schwarz, Peter, 78532 Tuttlingen (DE)

(57) **Zusammenfassung**

Hybridendoskop (10), insbesondere für sterile medizinische Anwendungen, wobei das Endoskop (10) eine Aufnahmevorrichtung (12) für ein Abbildungssystem (14) und ein Schaftrohr (16) umfasst, wobei die Aufnahmevorrichtung (12) in einem montierten Zustand an einem distalen Ende (55) des Schaftrohres (16) angeordnet ist. Dabei ist das Endoskop (10) in zumindest zwei Baugruppen (18, 20) unterteilt, wobei eine erste Baugruppe (18) zumindest die Aufnahmevorrichtung (12), das Abbildungssystem (14) und vorzugsweise eine Versorgungsleitung (22) des Abbildungssystems (14) als wiederverwendbare Teile umfasst und eine zweite Baugruppe (20) zumindest das Schaftrohr (16) als Teil zur Einmalverwendung umfasst, wobei die zwei Baugruppen (18, 20) mittels einer Schnittstelle (24) lösbar in eine Wirkungsverbindung bringbar sind.

## Beschreibung

Die vorliegende Erfindung betrifft ein Hybridendoskop mit teilweise wiederverwendbaren Teilen für sterile medizinische Anwendungen nach dem Oberbegriff des Anspruchs 1.

Endoskope, insbesondere für chirurgische Anwendungen, werden nach einer Operation zur Wiederverwendung gereinigt und desinfiziert. Die Reinigung umfasst dabei innere Leitungsführungen, Kanäle und Ventilanschlüsse und ein Abbildungssystem mit dazugehörigem mechanischen Stellmechanismus und elektrischen Anschlüssen. Die Desinfektion kann ein Autoklavierverfahren bei hohen Temperaturen von über 130°C und/oder die Verwendung von Chemikalien zur Desinfektion unterschiedlicher Infektionserreger wie Bakterien, Pilzen und Viren beinhalten. Um den widrigen Bedingungen während der Desinfektion standzuhalten, werden die Bauteile eines Endoskops vorzugsweise aus metallischen Materialien gefertigt, die im Vergleich zu Kunststoffmaterialien diesen Bedingungen länger standhalten und somit öfter wiederverwendet werden können. Insbesondere das Abbildungssystem mit elektronischen Anschlüssen ist deshalb üblicherweise in einem hermetisch abgedichteten Metallgehäuse eingefügt oder angeordnet.

Anschließend an ein Desinfektionsverfahren werden die gereinigten Teile getrocknet und bis zu einer nächsten Verwendung keimfrei, sowie von einer erneuten Kontamination geschützt, gelagert. Nach der DIN EN 16442 Norm ist die Lagerung zur Wiederverwendung von Endoskopen für medizinische Anwendung geregelt, wobei die Umgebungsbedingungen kontrolliert und die Endoskope in speziellen Schränken gelagert werden sollten.

Jedoch kann trotz einer zeit- und arbeitsaufwändigen, sowie vorschriftsgemäßen Desinfektion des Endoskops eine Keimanlagerung in besonders schwer zu reinigenden Teilen, wie den Leitungsführungen und langen Kanälen mit kleinem Durchmesser und den entsprechenden Anschlüssen, nicht vollständig ausgeschlossen werden.

Die regelmäßige Reinigung und die Herstellung von hermetisch abgedichteten Endoskopen sind nicht nur mit höheren Kosten verbunden, sondern auch die Funktionsweise des Endoskops ist durch die erhöhten konstruktiven Anforderungen stark eingeschränkt. Zum Beispiel ist ein Blickfeld des Abbildungssystems durch die Anordnung in einem starren Gehäuse mit Glasabdeckungen beschränkt und ein kostenintensives gekrümmtes Deckglas ist notwendig. Auch die Beleuchtung kann auf Grund von Streulicht an dem Deckglas nicht mit dem Abbildungssystem geschwenkt werden. Des Weiteren nimmt das Abbildungssystem durch die hermetische Abdichtung mehr Platz in einem Schaftrohr in Anspruch, sodass entweder ein Abbildungssystem mit geringer Auflösung oder alternativ ein nurmehr kleiner Arbeitskanal verwendet werden kann.

Alternativ zu desinfizierbaren Mehrwegendoskopen können steril verpackte Einwegendoskope verwendet werden, die nach dem Gebrauch vollständig entsorgt werden. Dies führt jedoch zu einer großen Menge an zu entsorgendem Müll, der in einer fachgerechten Entsorgung als Sondermüll behandelt werden muss.

Ein Schaft oder üblicherweise ein Schaftrohr des Endoskops kann starr oder flexibel oder abschnittsweise flexibel ausgeführt sein. Im Folgenden wird, wenn nicht genauer beschrieben, unter dem Schaftrohr ein starres und/oder flexibles Schaftrohr verstanden.

Die WO 2017/040692 A1 zeigt ein Endoskop mit einer kugelförmigen Rotationstrommel an einem langerstreckten starren Schaftrohr, insbesondere für die Verwendung als ein solches Einweginstrument, das in einer Lagergabel an einem distalen Ende des starren Schaftrohres drehbar gelagert ist, und mit einer Steuerleitung im Inneren des starren Schaftrohres rotiert werden kann. Die Rotationstrommel weist im Inneren ein Abbildungssystem auf und ist mit elektronischen Elementen im Inneren des starren Schaftrohres verbunden, wobei gleichzeitig eine Flüssigkeit durch das Innere des starren Schaftrohres geleitet werden kann. In dieser Ausführungsform eines Endoskops mit Rotationstrommel wird ein Großteil des starren Schaftrohres für die elektronischen Elemente des Abbildungssystems und der Steuerleitung aufgebraucht.

Insbesondere da die elektronischen Elemente innerhalb des Schaftrohres geführt sind und dort mit Flüssigkeit aus einem Operationsbereich in Kontakt kommen können, ist eine aggressive Desinfektion auch dieser Teile notwendig. Da diese Teile nicht hermetisch, zum Beispiel in einem Metallgehäuse, abgedichtet sind, ist eine wiederholte Reinigung der Teile nur bedingt ohne eine Beschädigung möglich. Ein Einweginstrument kann zwar die Herstellung und die Materialwahl vereinfachen, sowie ein Blickfeld des Abbildungssystems vergrößern, jedoch können insbesondere für den Austausch des Abbildungssystems, der Steuerleitung und der elektronischen Elemente, hohe Kosten anfallen.

Der vorliegenden Erfindung liegt die Aufgabe zugrunde, ein Endoskop vorzuschlagen, dass bei Vermeidung der aus dem Stand der Technik bekannten Probleme einen großen Arbeitskanal in Bezug auf den Innendurchmesser des Schaftrohres ermöglicht, für einen sterilen medizinischen Einsatz geeignet ist und gleichzeitig eine einfache, sichere und kostengünstige Reinigung ermöglicht.

Ferner besteht die Aufgabe darin ein Verfahren zum Bedienen/Verwenden des erfindungsgemäßen Endoskops anzugeben.

Diese Aufgabe wird hinsichtlich des Endoskops mit den Merkmalen des unabhängigen Anspruchs 1 gelöst und hinsichtlich des Verfahrens mit den Merkmalen des Anspruchs 10 gelöst.

Vorteilhafte Ausführungsformen sind Gegenstand der Unteransprüche.

Erfindungsgemäß wird ein Hybridendoskop, insbesondere für sterile medizinische Anwendungen, vorgeschlagen, wobei das Endoskop eine Aufnahmevorrichtung für ein Abbildungssystem und ein Schaftrohr umfasst, wobei die Aufnahmevorrichtung in einem montierten Zustand an einem distalen Ende des Schaftrohres angeordnet ist. Dabei ist das Endoskop in zumindest zwei Baugruppen unterteilt, wobei eine erste Baugruppe zumindest die Aufnahmevorrichtung, das Abbildungssystem und vorzugsweise eine Versorgungsleitung des Abbildungssystems als wiederverwendbare Teile umfasst und eine zweite Baugruppe zumindest das Schaftrohr als Teil zur Einmalverwendung umfasst, wobei die zwei Baugruppen mittels einer Schnittstelle lösbar in eine Wirkungsverbindung bringbar sind.

Dabei hat die Erfindung erstaunlicherweise erkannt, dass durch die Verwendung zumindest zweier getrennter Baugruppen die in der Herstellung mit hohen Kosten beaufschlagten Teile, insbesondere das Abbildungssystem mit hochwertigen optischen und elektronischen Komponenten, einfach von einem distalen Ende des Schaftrohres lösbar ist und so zur Wiederverwendung aufbereitet werden kann. Kostengünstige Teile und konstruktiv einfache, allerdings schwer zu reinigende Teile wie das Schaftrohr selbst sind zur Einmalverwendung als weitere zweite Baugruppe vorgesehen. Darunter fallen insbesondere Kanäle für eine Instrumentenführung und eine Fluidleitung und mechanische Gelenke mit zumindest einem Freiheitsgrad, die hermetisch schwer abdichtbar sind oder aufwändig mit einem Metallfaltenbalg überzogen werden müssten. Dadurch kann ein steriles Endoskop, insbesondere in Risikobereichen mit multiresistenten Keimen, dennoch kostengünstig und sogar ressourcenschonend eingesetzt werden. Außerdem verhindert insbesondere die getrennte Aufbereitung die Bildung von Schmutznestern, die bei einer Aufbereitung im zusammengebauten Zustand auftreten könnten. Des Weiteren kann ein hochwertiges Abbildungssystem für präzise Aufnahmen verwendet werden.

Zusätzlich zu den zwei Baugruppen wäre eine Aufteilung in weitere Bauteile oder Baugruppen denkbar, insbesondere für Abschnitte die nicht direkt in Kontakt mit dem Operationsbereich kommen und so einer weniger aufwendigen Aufbereitung unterliegen und unter Umständen auch mit einfachen und auswechselbaren Kunststoffüberzügen vor einer Verunreinigung schützbar sind oder geringere Anforderungen an die Dichtheit und die Desinfektion gestellt werden.

In einer besonders vorteilhaften Ausführungsform der Erfindung verläuft die Versorgungsleitung des Abbildungssystems auf einer Außenseite des Schaftrohres. Dadurch können die zwei Baugruppen besonders einfach voneinander getrennt und aufbereitet werden. Besonders durch die außenliegende Anordnung der Versorgungsleitung kann verhindert werden, dass sich Schmutznester im Inneren des Schaftrohres ansammeln können.

Vorzugsweise sind die Teile der ersten Baugruppe hermetisch abgedichtet und dadurch autoklavierbar. So kann das Abbildungssystem auch hochwertige Komponenten umfassen, die wiederverwendbar und auch bei einer aggressiven Reinigung/Desinfektion nicht beschädigt werden.

Es kann gemäß der Lehre der Erfindung vorgesehen sein, dass die Aufnahmevorrichtung der ersten Baugruppe aus einem Metall gefertigt ist und/oder das Schaftrohr der zweiten Baugruppe aus einem Kunststoff gefertigt ist und/oder die Versorgungsleitung mit einem autoklavierbaren Steckverbinder verbunden ist. Die metallische Aufnahmevorrichtung der ersten Baugruppe kann so den Reinigungsbedingungen in einem Autoklaven, insbesondere mit zusätzlicher chemikalischen Reinigung, über eine Vielzahl an Reinigungszyklen standhalten. Besonders bevorzugt kann das Schaftrohr der zweiten Baugruppe aus einem Kunststoff kostengünstig, zum Beispiel in einem Spritzgussverfahren, in großer Stückzahl herstellbar sein. Der Steckverbinder ist vorzugsweise als Anschluss zu einer Central Control Unit (CCU) vorgesehen, die nicht mit dem Operationsbereich in Kontakt steht oder von diesem kontaminiert wird.

Weiterhin ist bevorzugt, dass das Abbildungssystem einen elektronischen Bildaufnehmer und/oder eine Abbildungsoptik und/oder eine Beleuchtungseinrichtung, insbesondere mit LEDs, und/oder Sonderbaugruppen wie Laserprojektoren beinhaltet. Dadurch kann ein Operationsbereich untersucht und beobachtet werden. Insbesondere durch die Wiederverwendbarkeit der ersten Baugruppe können hochwertige Komponenten, insbesondere für den Bildaufnehmer, in der Aufnahmevorrichtung verbaut sein.

In einer bevorzugten Ausführungsform nimmt die Aufnahmevorrichtung der ersten Baugruppe das Abbildungssystem in einer Rotationstrommel auf und die zweite Baugruppe weist eine Schnittstelle in einer Lagergabel auf, um die Rotationstrommel rotierbar zu lagern.

Gemäß einer weiter bevorzugten Ausführungsform ist zumindest eine Steuerleitung bevorzugt auf den äußeren Umfang der Rotationstrommel entlang zumindest einer Aufwickelkurve aufwickelbar, um die Rotationstrommel zu schwenken.

Weiter bevorzugt ist die Versorgungsleitung des Abbildungssystems als zumindest eine Steuerleitung der Rotationstrommel ausgebildet, vorzugsweise als Flexleiterplatte oder als ein Kabel, für elektronische Schaltkreise im Inneren der Rotationstrommel. Die Versorgungsleitung kann elektronische Schaltkreise insbesondere einer elektronisch verstellbaren Abbildungsoptik und/oder des elektronischen Bildaufnehmers oder Sensors des Abbildungssystems und/oder der Beleuchtungseinrichtung, vorzugsweise mit LEDs, aufweisen.

Besonders bevorzugt ist die zumindest eine Steuerleitung in einer Aussparung, bevorzugt mit einem Schutzkragen, in der Außenfläche des Schaftrohres geführt, wobei die Aussparung parallel zu der Längsachse des Schaftrohres verläuft. Dadurch ist die zumindest eine Steuerleitung im montierten Zustand der ersten und zweiten Baugruppen platzsparend und formschlüssig auf der Außenseite des Schaftrohres geführt, um einerseits die zumindest eine Steuerleitung vor mechanischen äußeren Einflüssen zu schützen und um andererseits einem Verletzungsrisiko oder Einklemmen von Gewebe während einer Operation vorzubeugen.

Für die Steuerung der Rotationstrommel sind an der Rotationstrommel vorzugsweise Rückstellmittel angeordnet, bevorzugt eine Drehfeder in einer Lagerstelle der Rotationstrommel. Dabei wirkt die Drehfeder einem mechanischen Moment der auf Zug beanspruchten Steuerleitung entgegen, um eine geschwenkte Rotationstrommel in eine Ausgangsposition zu versetzen. Die Drehfeder wirkt als vorzugsweise derart einer Bedienung der Steuerleitung entgegen, dass die Steuerleitung für eine Rotation der Rotationstrommel nicht mit einer Druckkraft beaufschlagt werden muss und so eine flexible Steuerleitung verwendbar ist.

Alternativ oder zusätzlich kann die Rotationstrommel durch eine weitere Steuerleitung auf der gegenüberliegenden Seite zurückführbar sein und/oder mit einer formstabilen Steuerleitung, ähnlich eines Bowdenzuges, oder einem Art Steuerstab oder Zahnstange auch mittels einer Druckkraft bedienbar sein.

Weiter alternativ kann die weitere Steuerleitung vorzugsweise als ein Draht ausgebildet sein, wobei ein Rückstellmittel, vorzugsweise eine Feder, insbesondere eine Schraubenfeder, bevorzugt in einem Handgriff zur Rückstellung der Rotationstrommel befestigt ist.

Alternativ oder zusätzlich kann vorgesehen sein, dass die zweite Baugruppe als Schnittstelle ein rotierbares Gelenk mit Verstellmechanik zur Befestigung der Aufnahmevorrichtung aufweist. Im Vergleich zu der Rotationstrommel ist die Aufnahmevorrichtung vorzugsweise konstruktiv einfacher ausgestaltet, vorzugsweise nicht für eine Lagerstelle drehbar vorbereitet und kann vorzugsweise rückseitig, mit einer dem Abbildungssystem abgewandten Seite, mittels der Schnittstelle mit dem rotierbaren Gelenk verbindbar sein.

Die Schnittstelle kann eine form- und/oder kraft- und/oder stoffschlüssig Verbindung herstellen und bevorzugt als Steckverbindung oder Rastverbindung und/oder Schraubverbindung ausgebildet sein.

Vorzugsweise kann vorgesehen sein, dass der Arbeitskanal im Schaftrohr zumindest eine Fluidleitung, bevorzugt modular eine Luftleitung und/oder eine Flüssigkeitszufuhrleitung und eine Flüssigkeitsabfuhrleitung, aufweist. Insbesondere da das Abbildungssystem mit Versorgungsleitung nicht innerhalb des Schaftrohres angeordnet ist, können insbesondere mehrere Kanäle, wie die Luftleitung zusammen mit der Flüssigkeitszufuhrleitung und der Flüssigkeitsabfuhrleitung innerhalb des Schaftrohres vorgesehen sein. Eine Art Überschaft um das Schaftrohr zur Fluidleitung, der den Außendurchmesser des Schaftrohrs vergrößert, ist vorteilhafterweise nicht notwendig. Die Funktionalität der Fluidleitung kann die Optikreinigung und/oder ein Kühlen/Temperieren der Rotationstrommel und/oder ein Spülen und Insufflieren eines Fluids in einen Operationsbereich umfassen.

Vorteilhafterweise wird die Luftleitung in einem trockenen Operationsbereich eingesetzt, um die Rotationstrommel nach einer Optikreinigung zu trocknen und/oder die Rotationstrommel und der elektronischen Komponenten des Abbildungssystems zu kühlen.

In einer trockenen Umgebung des Endoskops weist der Arbeitskanal als zumindest eine Fluidleitung, vorzugsweise eine Luftleitung, eine Flüssigkeitszufuhrleitung und eine Flüssigkeitsabfuhrleitung auf. Die Optikreinigung des Endoskops findet in einem trockenen Operationsbereich vorzugsweise in einer Reinigungsstellung statt, in der die Rotationstrommel derart rotiert ist, dass das Blickfeld auf das distale Ende des Schaftrohrs und den Arbeitskanal ausgerichtet ist. In dieser Reinigungsstellung kann das Abbildungssystem mit einer Spülflüssigkeit aus der Flüssigkeitszufuhrleitung gespült werden, wobei mit der Flüssigkeitsabfuhrleitung die Spülflüssigkeit entfernbar ist und so ein Austritt an Spülflüssigkeit in einen trockenen Operationsbereich minimiert werden kann. Vorzugsweise mit der Luftleitung lässt sich die Spülflüssigkeit von dem Abbildungssystem entfernen, um eine Störung des Blickfelds des Abbildungssystems durch Tropfenbildung zu verhindern. Weiter bevorzugt kann die Flüssigkeitszufuhrleitung und vorzugsweise auch die Luftleitung als Spritzdüse ausgebildet sein, um die Reinigungswirkung zu verbessern.

Für einen wässrigen oder nassen Operationsbereich oder Umgebung des Endoskops ist vorzugsweise die Flüssigkeitszufuhrleitung und die Flüssigkeitsabfuhrleitung in dem Arbeitskanal angeordnet, um die Rotationstrommel rückseitig zu umspülen und zu temperieren, sowie den Operationsbereich zu spülen. Für die Spülung des medizinischen Operationsbereichs ist bevorzugt vorgesehen, dass das Schaftrohr an einem distalen Ende umfangsseitig Löcher und/oder eine, vorzugsweise schlitzförmige, Öffnung des Arbeitskanals aufweist, um die zumindest eine Fluidleitung, bevorzugt die Flüssigkeitsabfuhrleitung, mit dem Operationsbereich zu verbinden und um ein Spülfluid aus dem Operationsbereich zu entfernen. Insbesondere ist das Schaftrohr hinter der Rotationstrommel geöffnet, um einen freien Austritt des Spülfluids, insbesondere des Spülwassers, zu gewährleisten.

Vorzugsweise ist der Arbeitskanal der zweiten Baugruppe zur Aufnahme zumindest eines Instruments ausgebildet und/oder weist zumindest ein Instrument auf, um in dem Operationsbereich und insbesondere in dem Blickfeld des Abbildungssystems bevorzugt medizinische Operationen durchführen zu können. Dabei weist das zumindest eine Instrument an einem distalen Ende bevorzugt ein steuerbares Werkzeug, zum Beispiel ein scherenartiges Schneidewerkzeug oder Stanzwerkzeug, auf. Die Aufnahme des zumindest einen Instruments kann zusätzlich oder alternativ zu zumindest einer Fluidleitung vorgesehen sein. Durch die außenliegende Steuerleitung und insbesondere die außenliegende Versorgung des Abbildungssystems kann der relativ große Arbeitskanal für zumindest ein Instrument und gleichzeitig zumindest eine Fluidleitung verwendet werden.

Das zumindest eine Instrument kann von der ersten Baugruppe ebenfalls trennbar ausgeführt sein und wie die erste Baugruppe als eine weitere Baugruppe wiederverwendbar sein. Alternativ können auch Einweg-Instrumente verwendet werden, die nach dem Gebrauch nicht aufbereitet oder gereinigt werden.

In einer Weiterbildung, ist das Schaftrohr an einem oberen Abschnitt geöffnet, wobei die zumindest eine erste Steuerleitung an einem gegenüberliegenden unteren Abschnitt geführt ist, um vorzugsweise ein flexibles Instrument in eine Richtung senkrecht zu der Längsachse des Schaftrohrs und in den geöffneten oberen Abschnitt zu verstellen/biegen. Dabei ist der Arbeitskanal derart in dem Schaftrohr und hinter der Rotationstrommel angeordnet, dass das flexible Instrument an dem äußeren Umfang der Rotationstrommel entlang gleitet und durch den geöffneten oberen Abschnitt führbar ist. Dadurch kann das Instrument, insbesondere die funktionellen Teile und Werkzeuge an dem distalen Ende des Instruments, in einen Operationsbereich gebracht werden und gleichzeitig kann der Operationsbereich mit dem Abbildungssystem innerhalb der Rotationstrommel verfolgt werden.

Besonders für die Verwendung des Endoskops mit dem zumindest einen Instrument ist vorzugsweise vorgesehen, dass die Rotationstrommel um eine erste Rotationsachse schwenkbar an der Lagergabel befestigt ist und die Lagergabel wiederum schwenkbar um eine zweite Rotationsachse an dem Schaftrohr befestigt ist und der Arbeitskanal in einem geschwenkten Zustand der Lagergabel entlang der Längsachse geöffnet ist, um insbesondere das zumindest eine Instrument aus dem Arbeitskanal in einen Operationsbereich zu führen, wobei die Lagergabel vorzugsweise mit einer zweiten Steuerleitung steuerbar ist. Die zweite Steuerleitung ist vorzugsweise ebenfalls auf der Außenseite des Schaftrohres geführt, um den Arbeitskanal im Inneren des Schaftrohres nicht einzuschränken. Mit dieser Ausführungsform ist insbesondere ein starres Instrument parallel zu der Längsachse des Schaftrohrs in den Operationsbereich führbar. Des Weiteren ist mittels einer Rotation der Rotationstrommel um die zweite Rotationsachse ein vorzugsweise flexibles Instrument in eine Richtung senkrecht zu der Längsachse des Schaftrohrs biegbar.

Neben der Führung des zumindest einen Instruments, ermöglicht die schwenkbare Lagergabel vorteilhafterweise ein erweitertes Blickfeld des Abbildungssystems, auch zum Beispiel hinter Hindernisse, Ecken oder einen Blick entlang der Außenseite des Schaftrohrs. So ist zum Beispiel auch die Funktion der Führung der zumindest einen Steuerleitung entlang des Schaftrohres mittels des Abbildungssystems überprüfbar. Weiterhin kann mit dem geöffneten Arbeitskanal ein Spülen oder Absaugen von Fluiden aus dem Operationsbereich begünstigt werden.

Die schwenkbare Lagergabel kann bevorzugt der bereits erwähnten Schnittstelle mit rotierbaren Gelenk mit Verstellmechanik entsprechen, wobei eine schenkbare Aufnahmevorrichtung an dem Gelenk befestigbar ist.

Alternativ oder zusätzlich kann die Lagergabel auch mittels eines flexiblen oder elastisch verformbaren Elements mit dem Schaftrohr verbunden sein, wobei das flexible Element eine passive Rückstellkraft ausbildet, um die Lagergabel um die zweite Rotationsachse zu schwenken und den Arbeitskanal für das Instrument zu öffnen.

Vorzugsweise beträgt der Außendurchmesser des Schaftrohres 3mm bis 6mm, wobei die Rotationstrommel diesen Außendurchmesser nicht übersteigt. Solche Schaftrohre sind für eine Vielzahl nicht invasiver medizinischer Operationen geeignet.

In einer weiter bevorzugten Ausführungsform sind mehrere Steuerleitungen auf unterschiedlichen Aufwickelkurven entlang des Umfangs der Rotationstrommel aufwickelbar, insbesondere mit einem unterschiedlichen Abstand zu der Rotationsachse, um bei gleichem Verstellweg der Steuerleitung eine unterschiedliche Drehverstellung der Rotationstrommel auszuführen. Insbesondere der Verstellweg parallel zu der Längsachse des Schaftrohres und die unterschiedliche Drehverstellung kann zu vordefinierten Blickrichtungen des Abbildungssystems oder zu unterschiedlichen Rotationsgeschwindigkeiten führen, wobei bei gleichem Verstellweg der Steuerleitung die Drehverstellung mit kleiner werdendem Abstand zu der ersten Drehachse der Rotationstrommel abnimmt. Auch die für die Drehbewegung notwendige Zugkraft kann durch die unterschiedlichen Abstände eingestellt werden.

Besonders bevorzugt sind die unterschiedlichen Aufwickelkurven stufenweise entlang der ersten Rotationsachse auf dem Umfang der Rotationstrommel angeordnet, wobei die Aufwickelkurven vorzugsweise mit kleiner werdendem Abstand zu der ersten Rotationsachse in Richtung der äußeren Seiten der Rotationstrommel angeordnet sind.

Die Erfindung betrifft auch ein Verfahren zum Bedienen und Reinigen eines voran beschriebenen Endoskops mit Rotationstrommel, insbesondere des Blickfelds des Abbildungssystems, wobei die Rotationstrommel in einem ersten Schritt von einer Beobachtungsstellung eines Operationsbereichs in eine Reinigungsstellung, in der das Blickfeld auf das distale Ende des Schaftrohrs und den Arbeitskanal ausgerichtet ist, rotiert wird und wobei das im folgende beschriebene Reinigungsverfahren insbesondere für Endoskope in einer trockenen Umgebung oder einem trockenen Operationsbereich geeignet ist, da ein Austritt an Spülflüssigkeit in einen Operationsbereich minimiert werden kann. In einem zweiten Schritt wird das Abbildungssystem und/oder die Abbildungsoptik und/oder die Beleuchtungseinrichtung, insbesondere in dem Blickfeld, mit einem vorzugsweise wässrigen Spülfluid aus zumindest einer Fluidleitung, vorzugsweise einer Flüssigkeitszufuhrleitung mit einer Spritzdüse, gespült. Vorzugsweise kann das wässrige Spülfluid mit einer weiteren zweiten Fluidleitung, vorzugsweise eine Flüssigkeitsabfuhrleitung, abgesaugt werden, um den Austritt an Spülflüssigkeit in einen Operationsbereich zu minimieren. Dabei kann die Rotationstrommel geschwenkt werden, um das gesamte Blickfeld zu reinigen. In einem bevorzugten dritten Schritt wird das vorzugsweise wässrige Spülfluid bevorzugt mit einer Luftströmung aus einer Luftleitung entfernt und so das Blickfeld des Abbildungssystems getrocknet. Dieser Schritt ist insbesondere notwendig, falls das Endoskop in einem trockenen Operationsbereich eingesetzt wird. In einem vierten Schritt wird die Rotationstrommel zurück in die Beobachtungsstellung, vorzugsweise die Beobachtungsstellung aus dem ersten Schritt, rotiert.

Die Erfindung betrifft weiterhin ein Verfahren zum Bedienen eines voran beschriebenen Endoskops mit Rotationstrommel und einer schwenkbaren Lagergabel, wobei in einem ersten Schritt das Schaftrohr, zumindest das distale Ende des Schaftrohres, in einen Operationsbereich geführt und in einem zweiten Schritt die Lagergabel um eine zweite Rotationsachse geschwenkt wird, bis zumindest die Rotationstrommel den Arbeitskanal entlang einer Längsachse des Schaftrohres freigibt. In einem dritten Schritt wird ein Instrument aus dem Arbeitskanal herausgeführt, insbesondere in den medizinischen Operationsbereich hinein. Anschließend und in einem bevorzugten vierten Schritt wird die Rotationstrommel mittels der Steuerleitung um eine erste Rotationsachse geschwenkt, um das Instrument mit einem Blickfeld des Abbildungssystems im Operationsbereich zu verfolgen und für eine Bedienperson abzubilden. In einem fünften Schritt kann die Lagergabel um eine zweite Rotationsachse geschwenkt werden, um das Instrument zu bewegen oder zu verbiegen und in einen definierten Operationsbereich auszurichten.

Vorzugsweise weist die zweite Baugruppe auf einer Außenfläche des Schaftrohres einen verschiebbaren Griff auf, der parallel zu der Längsachse des Schaftrohres verschiebbar ist und wobei der Griff eine Aussparung zur Aufnahme eines Mitnahmezapfens an der Steuerleitung der ersten Baugruppe aufweist.

Die Erfindung betrifft auch ein Verfahren zur Verwendung eines bereits beschriebenen Hybridendoskops, wobei zumindest die zweite Baugruppe nach der Herstellung in einem sterilen Zustand für eine sterile Lagerung verpackt wird und als Einwegbauteil markiert wird. Anschließend werden die zwei Baugruppen in einem sterilen Zustand unmittelbar vor dem Einsatz, insbesondere für eine medizinische Operation, montiert und danach wieder demontiert. Nach der Demontage wird die erste Baugruppe für einen erneuten sterilen Einsatz in einem Autoklav und/oder mit einer chemischen Flüssigkeit aufbereitet. Die zweite Baugruppe wird einmalig verwendet, entsorgt und/oder mit einer Markierung versehen, die die vorangegangene Verwendung anzeigt und vorzugsweise eine Bedienperson an einer weiteren Montage mit einer ersten Baugruppe hindert. Zum Beispiel wäre es denkbar, dass bei der Demontage an einer Sollbruchstelle, vorzugsweise innerhalb der Schnittstelle, die zweite Baugruppe derart beschädigt wird, dass eine erneute Montage ausgeschlossen ist.

Weitere Vorteile und Einzelheiten der Erfindung ergeben sich aus der nachfolgenden Beschreibung bevorzugter Ausführungsformen der Erfindung sowie anhand von lediglich schematischen Zeichnungen.

Es zeigen:
- Fig. 1a:: eine Seitenansicht eines Hybridendoskops in einem montierten Zustand mit einer Rotationstrommel, die in einem Schaftrohr gelagert ist,
- Fig. 1b:: eine Seitenansicht gemäß der Fig. 1a einer ersten Baugruppe mit Rotationstrommel und gestrichelt dargestelltem Abbildungssystem,
- Fig. 1c:: eine Seitenansicht gemäß der Fig. 1a einer zweiten Baugruppe mit dem Schaftrohr,
- Fig. 2a:: eine Seitenansicht des Hybridendoskops in einem montierten Zustand mit einer Aufnahmevorrichtung, dem Schaftrohr und einem rotierbaren Gelenk,
- Fig. 2b:: eine Seitenansicht gemäß der Fig. 2a der ersten Baugruppe,
- Fig. 2c:: eine Seitenansicht gemäß der Fig. 2a des Schaftrohrs mit rotierbaren Gelenk.

Gleiche Elemente beziehungsweise Elemente mit gleicher Funktion sind in den Figuren mit den gleichen Bezugsziffern versehen.

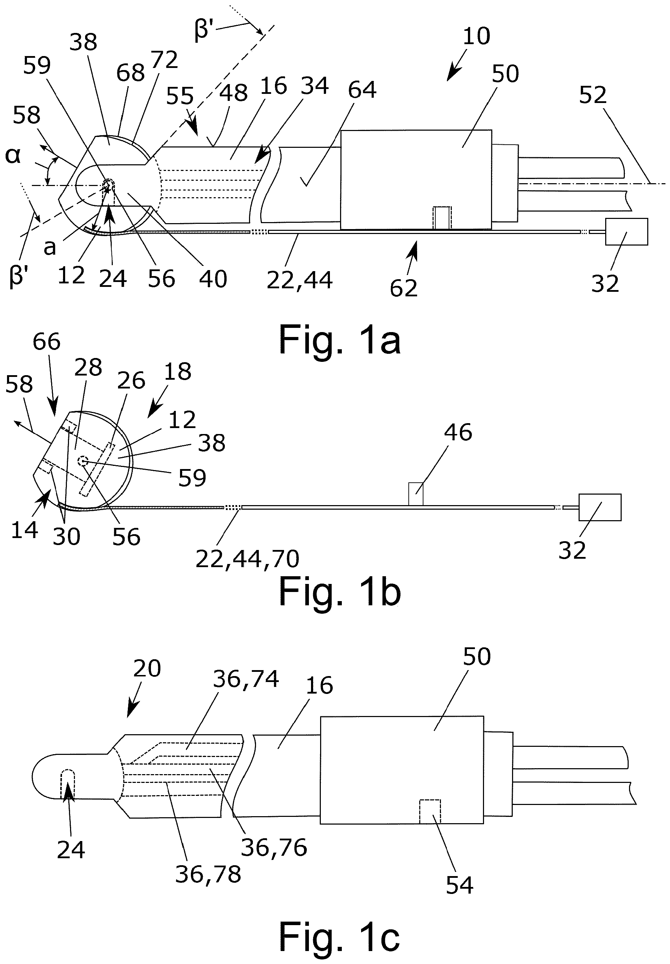

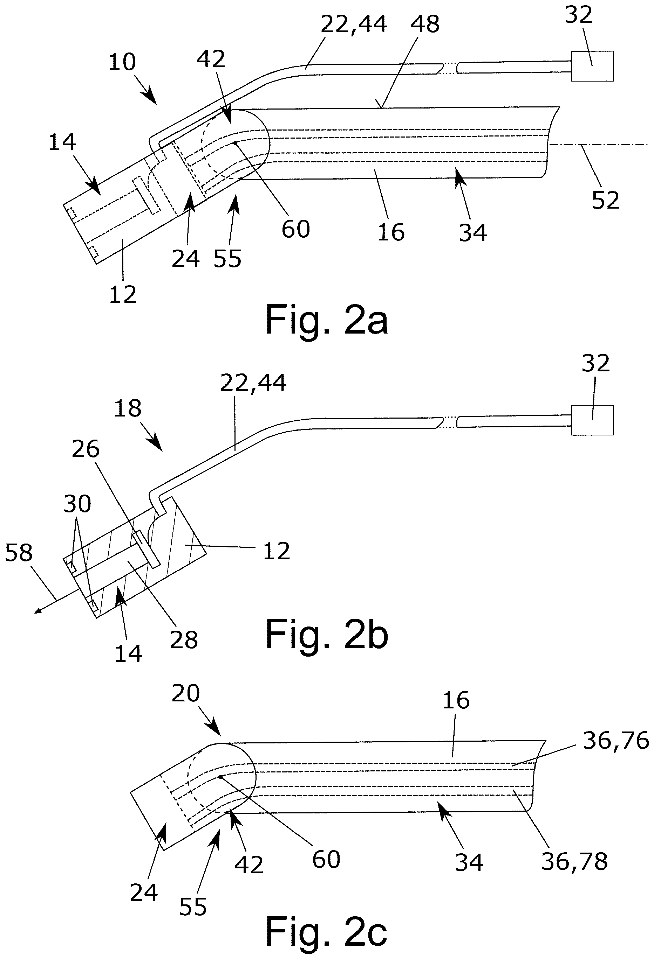

Die Fig. 1a und die Fig. 2a zeigen jeweils ein Hybridendoskop 10, insbesondere für eine sterile medizinische Anwendung, wobei das Endoskop 10 eine Aufnahmevorrichtung 12 für ein Abbildungssystem 14 und ein Schaftrohr 16 umfasst. Dabei ist das Hybridendoskop 10 in der Fig. 1a und der Fig. 2a in einem montierten Zustand dargestellt, wobei die Aufnahmevorrichtung 12 an einem distalen Ende 55 des Schaftrohres 16 angeordnet ist. Das Endoskop 10 ist in zwei Baugruppen 18, 20 unterteilt, die jeweils für sich genommen in der Fig. 1b und Fig. 1c, sowie der Fig. 2b und der Fig. 2c dargestellt sind. Die Fig. 1b und die Fig. 2b zeigen jeweils eine erste Baugruppe 18, die zumindest die Aufnahmevorrichtung 12 und das Abbildungssystem 14, sowie vorzugsweise eine Versorgungsleitung 22 des Abbildungssystems 14 als wiederverwendbare Teile umfasst. Die Fig. 1c und die Fig. 2c zeigen jeweils eine zweite Baugruppe 20 die zumindest das Schaftrohr 16 als Teil zur Einmalverwendung umfasst. Wie im montierten Zustand in den Fig. 1a und Fig. 2a dargestellt, sind die zwei Baugruppen 18, 20 mittels einer Schnittstelle 24 lösbar in eine Wirkverbindung bringbar.

Die Fig. 1a und die Fig. 2a zeigen, dass die Versorgungsleitung 22 des Abbildungssystems 14 vorzugsweise auf einer Außenseite 48 des Schaftrohrs 16 verläuft. Dadurch wird ein Arbeitskanal 34 im Inneren des Schaftrohres 16 nicht durch diese Versorgungsleitung 22 eingeschränkt. Des Weiteren sind durch die außenliegende Führung der Versorgungsleitung 22 die zwei Baugruppen 18, 20 besonders einfach montierbar oder demontierbar, insbesondere einfacher lösbar als eine Versorgungsleitung 22 die komplex im Inneren des Schaftrohres 16 geführt ist.

Vorzugsweise sind die Teile der ersten Baugruppe 18, insbesondere der Aufnahmevorrichtung 12 hermetisch abgedichtet und dadurch autoklavierbar.

Besonders bevorzugt ist die Aufnahmevorrichtung 12 der ersten Baugruppe 18 aus einem Metall gefertigt und bevorzugt das Schaftrohr der zweiten Baugruppe 20 aus einem Kunststoff gefertigt und vorzugsweise die Versorgungsleitung 22 mit einem autoklavierbaren Steckverbinder 32 verbunden. Dieser Steckverbinder 32 ist vorzugsweise mit einem Anschluss verbunden, der mit dem Operationsbereich nicht in Kontakt steht und weniger aufwändigen Reinigungsprozessen untersteht.

Wie in der Fig. 1b und der Fig. 2b gezeigt, kann das Abbildungssystem 14 vorzugsweise einen elektronischen Bildaufnehmer 26, eine Abbildungsoptik 28 und eine Beleuchtungseinrichtung 30, insbesondere mit LEDs umfassen. Die Abbildungsoptik 28 des Abbildungssystems 14 ist in eine Blickrichtung 58 ausgerichtet, um in dieser Blickrichtung 58 ein Bild auf dem Bildaufnehmer 26 zu erfassen.

Des Weiteren können in der Aufnahmevorrichtung 12 zusätzlich oder alternativ zu dem Abbildungssystem 14 Sonderbaugruppen wie Laserprojektoren angeordnet sein.

Besonders bevorzugt ist die Aufnahmevorrichtung 12 der ersten Baugruppe 18 als Rotationstrommel 38, gemäß der Fig. 1a und der Fig. 1b, ausgeführt, welche zumindest das Abbildungssystem 14 aufnimmt. Die Rotationstrommel 38 kann, gemäß der Fig. 1a, in einer Schnittstelle 24 einer Lagergabel 40 am distalen Ende 55 des Schaftrohres 16 der zweiten Baugruppe 20 montiert, insbesondere in dieser Lagergabel 40 rotierbar gelagert sein, wobei die Versorgungsleitung 22 des Abbildungssystems 14 als Steuerleitung 22, 44 der Rotationstrommel 38 dient und die Rotationstrommel 38 vorzugsweise mit Lagerstiften 56 in die Schnittstelle 24, vorzugsweise formschlüssig, einschiebbar und einrastbar ist.

Eine erste Rotationsachse 59 der Rotationstrommel 38 verläuft etwa quer zu einer Längsachse 52 des Schaftrohrs 16, wobei das Endoskop 10, wie in der Fig. 1a dargestellt, zumindest eine, vorzugsweise eine, Steuerleitung 22, 44 auf einer Unterseite 62 des Schaftrohres 16 aufweist. Diese zumindest eine Steuerleitung 22, 44 ist auf einer Außenseite 48 des Schaftrohrs 16 geführt und mit einem Hebelabstand a zu der ersten Rotationsachse 58 an der Rotationstrommel 38 befestigt. Durch eine Linearbewegung der zumindest einen Steuerleitung 22, 44 parallel zu der Längsachse 52 des Schaftrohrs 16 ist die Rotationstrommel 38 rotierbar.

Die Rotationstrommel 38 ist vorzugsweise kugel- oder zylinderförmig ausgeführt, wobei die Rotationstrommel 38 bevorzugt senkrecht zu einer Blickrichtung 58 des Abbildungssystems 14 abgeflacht ist, um in einem abgeflachten Bereich 66, gemäß der Fig. 1b, die Abbildungsoptik 28 und die Beleuchtungseinrichtung 30, vorzugsweise als zwei LEDs, aufzunehmen.

Die zumindest eine Steuerleitung 22, 44 ist vorzugsweise auf zumindest eine Aufwickelkurve 68 entlang des äußeren Umfangs der Rotationstrommel 38 aufwickelbar. Besonders bevorzugt ist die zumindest eine Steuerleitung 22, 44 derart aufwickelbar, dass die Rotationstrommel 38 von einer Blickrichtung 58 entlang der Längsachse 52 des Schaftrohres 16 um zumindest einen Winkel α von 0° bis 180° drehbar ist und so die Blickrichtung 58 in das Innere des Schaftrohres 16 und einen Arbeitskanal 34 ausrichtbar ist. Der Blickwinkel β des Abbildungssystems 14 beträgt vorzugsweise 50° bis 70°, besonders bevorzugt 60°. Dadurch beträgt ein bevorzugter Beobachtungsbereich β', indem das Abbildungssystem 14 den Operationsbereich durch Schwenken der Rotationstrommel 38 um einen Winkel α aufnehmen kann, zwischen -35° und 215°, besonders bevorzugt zwischen -30° und 210°, wobei der Winkel des Beobachtungsbereich β', wie auch der Winkel α sich auf eine 0° Richtung entlang der Längsachse 52 des Schaftohres 16 beziehen. Für das Schwenken der Rotationstrommel 38 ist die zumindest eine Steuerleitung 22, 44 zumindest teilweise flexibel ausgeführt, um diese auf die zumindest eine Aufwickelkurve 68 der Rotationstrommel 20 aufzuwickeln. Dabei kann die zumindest eine Aufwickelkurve 68 entlang des äußeren Umfangs vorzugsweise kreisförmig oder oval ausgeführt sein.

Besonders bevorzugt bildet die zumindest eine Steuerleitung 22, 44 der Rotationstrommel 38 eine Versorgungsleitung 22 für das Abbildungssystem 14 aus, das vorzugsweise als ein in der Fig. 1a oder Fig. 1b, dargestellte Flexleiterplatte 70 ausgeführt ist, um elektronische Schaltkreise im Inneren der Rotationstrommel 38 zu versorgen. Insbesondere bei der Verwendung der Flexleiterplatte 70 als Steuerleitung 22, 44 ist die entsprechende Aufwickelkurve 68 derart ausgebildet, dass der Biegeradius größer als 0,5mm ist. Alternativ kann das Abbildungssystem 14 auch mit einem Kabel elektrisch versorgt sein.

Die zumindest eine Steuerleitung 22, 44 ist vorzugsweise in einer Vertiefung 72, gemäß der Fig. 1a und der Fig. 1b, auf dem Umfang der Rotationstrommel 38 formschlüssig geführt, damit diese geschützt ist, gleichzeitig der Außendurchmesser der Rotationstrommel 38 nicht weiter vergrößert wird und so auch eine Verletzung von Gewebe in einem Operationsbereich reduziert werden kann.

Zusätzlich oder alternativ weist die zweite Baugruppe 20, gemäß der Fig. 2a und der Fig. 2c, an dem distalen Ende 55 des Schaftrohrs 16 ein rotierbares Gelenk 42 zur Befestigung der Aufnahmevorrichtung 12 auf. Vorzugsweise kann die in der Fig. 2b dargestellte Aufnahmevorrichtung 12 mit Versorgungsleitung 22 an dem rotierbaren Gelenk 42 befestigt sein, wobei die Versorgungsleitung 22 als Steuerleitung 22, 44 des rotierbaren Gelenks 42 dient.

Dieses rotierbare Gelenk 42 kann auch zusätzlich zu der Ausführungsform in der Fig. 1a vorgesehen sein, wobei die Lagergabel 40 als rotierbares Gelenk 42 ausgeführt ist und die Rotationstrommel 38 um eine erste Rotationsachse 59 und eine zweite Rotationsachse 60 des rotierbaren Gelenks 42 schwenkbar ist.

Vorzugsweise weist das Schaftrohr 16 der zweiten Baugruppe 20 zumindest einen Arbeitskanal 34 auf. Dieser Arbeitskanal 34, gemäß der Fig. 1c oder der Fig. 2c, kann zumindest eine Fluidleitung 36 aufweisen. Alternativ oder zusätzlich kann in dem Arbeitskanal 34 ein nicht dargestelltes Instrument geführt werden.

In dem Arbeitskanal 34 des Schaftrohres 16 sind vorzugsweise eine Luftleitung 36, 74 und/oder bevorzugt eine Flüssigkeitszufuhrleitung 36, 76 und eine Flüssigkeitsabfuhrleitung 36, 78 angeordnet. Die Luftleitung 36, 74 und die Flüssigkeitsleitungen 36, 76, 78 sind in der Fig. 1c und der Fig. 2c dargestellt. In einer Beobachtungsstellung, gemäß der Fig. 1a und Fig. 2a, können die Luft- und/oder Flüssigkeitsleitung 36 zur rückseitigen Kühlung des Abbildungssystems 14 verwendet werden.

Insbesondere für die Reinigung des Abbildungssystems 22 von Endoskopen 10 in einem trockenen Operationsbereich, ist bevorzugt die Rotationstrommel 38 in eine Reinigungsstellung verdrehbar, wobei die Rotationstrommel 38 mit der Blickrichtung 58 in Richtung des Arbeitskanals 34 geneigt ist. In dieser Reinigungsstellung ist vorzugsweise mit einer Luftleitung 36, 74 in Kombination mit der Flüssigkeitszufuhrleitung 36, 76 eine Verschmutzung von dem abgeflachten Bereich 66 der Rotationstrommel 38 spülbar und damit ein Blickfeld des Abbildungssystems 14 reinigbar, wobei die Luftleitung 36, 74 vorzugsweise dafür verwendet wird, eine Spülflüssigkeit von dem Abbildungssystem 14 und dem abgeflachten Bereich 66 zu entfernen. Vorzugsweise ist mit einer Flüssigkeitsabfuhrleitung 36, 78 die Spülflüssigkeit entfernbar, wodurch ein Austritt an Spülflüssigkeit in einen trockenen Operationsbereich minimiert werden kann.

Vorzugsweise für Endoskope 10 mit nasser Umgebungsbedingung oder in einem nassem Operationsbereich kann zusätzlich zu der rückseitigen Kühlung und der Reinigung der Rotationstrommel 38 oder der Aufnahmevorrichtung 12 die zumindest eine Fluidleitung 36 dafür verwendet werden einen Operationsbereich zu spülen, insbesondere um den Operationsbereich in der Blickrichtung 58 des Abbildungssystems 14 in einer Beobachtungsstellung zu spülen. Für eine medizinische Anwendung wird dafür vorzugsweise ein steriles Fluid verwendet, wie aufbereitete Luft, Sauerstoff oder eine physiologische Salzlösung als Flüssigkeit. Vorzugsweise wird jedoch ein wässriges Spülfluid verwendet. Dabei kann das Abbildungssystem 22 gleichzeitig mit dem Spülen des Operationsbereichs reinigbar sein, ohne dabei die Rotationstrommel 38 in eine Reinigungsstellung schwenken zu müssen.

In einer bevorzugten Ausführungsform, gemäß der Fig. 1a und Fig. 1c, weist das Schaftrohr 16 der zweiten Baugruppe 20 auf einer Außenfläche 48 einen verschiebbaren Griff 50 auf. Der Griff 50 ist parallel zu der Längsachse 52 des Schaftrohrs 16 verschiebbar und weist vorzugsweise eine Aussparung 54 zur Aufnahme eines Mitnahmezapfens 46 einer Steuerleitung 22, 44, insbesondere der Versorgungsleitung 22 des Abbildungssystems 14, der ersten Baugruppe 18 auf. Durch ein Verschieben des Griffs 50 ist die Rotationstrommel 38, gemäß der Fig. 1a, oder auch die Aufnahmevorrichtung 12, gemäß der Fig. 2a, schwenkbar.

Die Erfindung betrifft auch ein Verfahren zum Betrieb des voran genannten Hybridendoskops, wobei die zwei Baugruppen 18, 20, wie in der Fig. 1a und Fig. 2a dargestellt, in einem sterilen Zustand unmittelbar vor dem Einsatz, insbesondere für eine medizinische Operation, montiert werden und anschließend die erste Baugruppe 18, gemäß der Fig. 1b und der Fig. 2b, für einen erneuten sterilen Einsatz in einem Autoklav und/oder mit einer chemischen Flüssigkeit aufbereitet werden und die zweite Baugruppe 20, gemäß der Fig. 1c und der Fig. 2c, einmal verwendet wird und deshalb entsorgt wird.

Das soweit beschriebene Hybridendoskop 10 kann in vielfältiger Art und Weise abgewandelt oder modifiziert werden, ohne vom Erfindungsgedanken abzuweichen. So ist es zum Beispiel denkbar, die zwei Baugruppen 18, 20 zu markieren, insbesondere mit einem Barcode oder einer elektronischen Schnittstelle, sodass durch ein analoges oder elektronisches Auslesen, zum Beispiel sobald das Abbildungssystem 14 elektronisch mit einem Steuergerät verbunden ist, erkannt werden kann, ob eines der Baugruppen 18, 20 steril oder bereits verwendet oder gereinigt wurde, um einen erneuten Gebrauch eines bereits verwendeten und nicht sterilen Endoskops 10 zu verhindern.

### Bezugszeichenliste

- 10: Endoskop
- 12: Aufnahmevorrichtung
- 14: Abbildungssystem
- 16: Schaftrohr
- 18: erste Baugruppe des Endoskops
- 20: zweite Baugruppe des Endoskops
- 22: Versorgungsleitung des Abbildungssystems
- 24: Schnittstelle
- 26: elektronischer Bildaufnehmer
- 28: Abbildungsoptik
- 30: Beleuchtungseinrichtung
- 32: autoklavierbarer Steckverbinder
- 34: Arbeitskanal
- 36: Fluidleitung
- 38: Rotationstrommel
- 40: Lagergabel
- 42: rotierbares Gelenk
- 44: Steuerleitung
- 46: Mitnahmezapfens
- 48: Außenfläche/Außenseite des Schaftrohres
- 50: verschiebbarer Griff
- 52: Längsachse des Schaftrohres
- 54: Aussparung im verschiebbaren Griff
- 55: distale Ende des Schaftrohrs
- 56: Lagerstifte der Rotationstrommel
- 58: Blickrichtung
- 59: erste Rotationsachse
- 60: zweite Rotationsachse
- 62: Unterseite des Schaftrohrs
- 66: abgeflachter Bereich der Rotationstrommel
- 68: Aufwickelkurve
- 70: Flexleiterplatte
- 72: Vertiefung
- 74: Luftleitung
- 76: Flüssigkeitszufuhrleitung
- 78: Flüssigkeitsabfuhrleitung
- a: Abstand zw. der ersten Rotationsachse und der Aufwickelkurve
- α: Winkel der Blickrichtung des Abbildungssystems
- β': Beobachtungsbereich

## Patentansprüche

1. Hybridendoskop (10), insbesondere für sterile medizinische Anwendungen, wobei das Endoskop (10) eine Aufnahmevorrichtung (12) für ein Abbildungssystem (14) und ein Schaftrohr (16) umfasst, wobei die Aufnahmevorrichtung (12) in einem montierten Zustand an einem distalen Ende (55) des Schaftrohres (16) angeordnet ist,
**dadurch gekennzeichnet,**
**dass** das Endoskop (10) in zumindest zwei Baugruppen (18, 20) unterteilt ist, wobei eine erste Baugruppe (18) zumindest die Aufnahmevorrichtung (12), das Abbildungssystem (14) und vorzugsweise eine Versorgungsleitung (22) des Abbildungssystems (14) als wiederverwendbare Teile umfasst und eine zweite Baugruppe (20) zumindest das Schaftrohr (16) als Teil zur Einmalverwendung umfasst, wobei die zwei Baugruppen (18, 20) mittels einer Schnittstelle (24) lösbar in eine Wirkungsverbindung bringbar sind.

2. Hybridendoskop nach dem Anspruch 1,
**dadurch gekennzeichnet,**
**dass** die Versorgungsleitung (22) des Abbildungssystems (14) auf einer Außenseite (48) des Schaftrohres (16) verläuft.

3. Hybridendoskop nach dem Anspruch 1 oder 2,
**dadurch gekennzeichnet,**
**dass** die Teile der ersten Baugruppe (18) hermetisch abgedichtet und dadurch autoklavierbar sind.

4. Hybridendoskop nach einem der Ansprüche 1 bis 3,
**dadurch gekennzeichnet,**
**dass** die Aufnahmevorrichtung (12) der ersten Baugruppe (18) aus einem Metall gefertigt ist und/oder das Schaftrohr (16) der zweiten Baugruppe (20) aus einem Kunststoff gefertigt ist und/oder die Versorgungsleitung (22) mit einem autoklavierbaren Steckverbinder (32) verbunden ist.

5. Hybridendoskop nach einem der Ansprüche 1 bis 4,
**dadurch gekennzeichnet,**
**dass** das Abbildungssystem (14) einen elektronischen Bildaufnehmer (26) und/oder eine Abbildungsoptik (28) und/oder eine Beleuchtungseinrichtung (30), insbesondere mit LEDs, und/oder Sonderbaugruppen wie Laserprojektoren beinhaltet.

6. Hybridendoskop nach einem der Ansprüche 1 bis 5,
**dadurch gekennzeichnet,**
**dass** die Aufnahmevorrichtung (12) der ersten Baugruppe (18) das Abbildungssystem (14) in einer Rotationstrommel (38) aufnimmt und die zweite Baugruppe (20) eine Schnittstelle (24) in einer Lagergabel (40) aufweist, um die Rotationstrommel (38) rotierbar zu lagern, wobei die Versorgungsleitung (22) des Abbildungssystems (14) als zumindest eine Steuerleitung (22, 44) der Rotationstrommel (38) dient.

7. Hybridendoskop nach einem der Ansprüche 1 bis 6,
**dadurch gekennzeichnet,**
**dass** die zweite Baugruppe (20) als Schnittstelle (24) ein rotierbares Gelenk (42) mit Verstellmechanik zur Befestigung der Aufnahmevorrichtung (12) aufweist.

8. Hybridendoskop nach einem der Ansprüche 1 bis 7,
**dadurch gekennzeichnet,**
**dass** das Schaftrohr (16) der zweiten Baugruppe (20) zumindest einen Arbeitskanal (34) zur Führung von zumindest einer Fluidleitung (36) und/oder zumindest eines Instruments aufweist.

9. Hybridendoskop nach einem der Ansprüche 6 bis 8,
**dadurch gekennzeichnet,**
**dass** die zweite Baugruppe (20) auf einer Außenfläche (48) des Schaftrohres (16) einen verschiebbaren Griff (50) aufweist, der parallel zu der Längsachse (52) des Schaftrohres (16) verschiebbar ist und wobei der Griff (50) eine Aussparung (54) zur Aufnahme eines Mitnahmezapfens (46) an der zumindest einen Steuerleitung (22, 44) der ersten Baugruppe (18) aufweist.

10. Verfahren zur Verwendung eines Hybridendoskops (10), bevorzugt nach einem der Ansprüche 1 bis 9, mit den folgenden Schritten:
- Herstellung und anschließende sterile Verpackung zumindest einer zweiten Baugruppe (20), bevorzugt umfassend eine Abbildungssystem (14), für eine sterile Lagerung,
- Montage einer ersten Baugruppe (18), bevorzugt umfassend ein Schaftrohr (16), an der zweiten Baugruppe (20) in einem sterilen Zustand, insbesondere unmittelbar vor dem Einsatz, insbesondere für eine medizinische Operation,
- Demontage der zwei Baugruppen (18, 20), bevorzugt nach Abschluss des Einsatzes,
- Aufbereitung der ersten Baugruppe (18) für einen erneuten sterilen Einsatz in einem Autoklav und/oder mit einer chemischen Flüssigkeit,
- Entsorgung der zweiten Baugruppe (20) und/oder Markierung nach einer Verwendung.
